(19) Europäisches Patentamt
European Patent Office
Office européen des brevets

(11) Publication number: **0 253 571**
**B1**

# (12) EUROPEAN PATENT SPECIFICATION

(45) Date of publication of the patent specification: **23.08.89**

(51) Int. Cl.⁴: **C 07 B 55/00,** C 07 B 57/00, C 07 C 85/00, C 07 D 243/00, C 07 D 243/26

(21) Application number: **87306067.7**

(22) Date of filling: **09.07.87**

(54) **Process for resolution and race mization of amines with acidic alpha-hydrogens.**

(30) Priority: **14.07.86 US 885047**

(43) Date of publication of application:
**20.01.88 Bulletin 88/3**

(45) Publication of the grant of the patent:
**23.08.89 Bulletin 89/34**

(84) Designated Contracting States:
**CH DE FR GB IT LI NL**

(56) References cited:
**EP-A-0 030 871**
**EP-A-0 119 804**
**EP-A-0 167 919**
**CH-A-601 263**
**GB-A-1 574 641**
**US-A-4 215 223**

**E.L.ELIEL "Stereochemie der Kohlenstoffverbindungen",1966 VERLAG CHEMIE Weinheim S. 59-65**

(73) Proprietor: **MERCK & CO. INC., 126, East Lincoln Avenue P.O. Box 2000, Rahway New Jersey 07065- 0900 (US)**

(72) Inventor: **Reider, Paul J., 621 Limball Avenue, Westfield New Jersey 07090 (US)**
Inventor: **Grabowski, Edward J.J., 741 Marcellus Drive, Westfield New Jersey 07090 (US)**

(74) Representative: **Hesketh, Alan, Dr., European Patent Department Merck & Co., Inc. Terlings Park Eastwick Road, Harlow Essex, CM20 2QR (GB)**

**EP 0 253 571 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convension).

## Description

The instant invention is directed to a one-pot process for the resolution-racemization of primary amines with α-hydrogens via selective crystallization of diastereomeric chiral sulfonic acid salts and the subsequent <u>in situ</u> racemization of the other enantiomer by the catalytic addition of aromatic aldehydes, which permits the stereospecific synthesis of the desired isomer.

## Background of the Invention

Processes for resolution-racemization of phenyl glycine are known (Clark <u>et al., J. C. S. Perkin I</u>, 475 - 481, 1976). However, the known processes require stoichiometric amounts of both a weak, chiral acid and an aromatic aldehyde, which severely limits the processes to applications involving amines considered capable of forming tartrate salts.

It was therefore an object of this invention to develop an economical, one-pot process for the resolution-racemization of weakly-to-moderately basic organic amines containing α-hydrogens employing only a catalytic amount of an aldehyde. It was a further object of this invention to develop an efficient process for the preparation and isolation of the optically-pure desired isomer of a chiral sulfonic acid salt.

## Description of the Invention

The present invention is directed to a one-pot, high yield process for the resolution-racemization of primary amines with acidic α-hydrogens, the pKa's of which are less than or equal to 15, which comprises adding 0.86 - 0.94 mole equivalents of a chiral sulfonic acid in an appropriate organic solvent and 1 to 5 mole equivalents of water to a solution of a racemic mixture of a primary amine with an α-hydrogen to create a resolution mixture; seeding the resolution mixture with a crystalline diastereomeric salt derived from the primary amine and the chiral sulfonic acid; adding a catalytic amount of an aromatic aldehyde to the resolution mixture; and filtering the resolution mixture to isolate the optically-pure diastereomeric salt of the primary amine with an α-hydrogen.

Examples of chiral sulfonic acids useful in this invention are camphorsulfonic acid, specifically (1S)-(+)-10-camphorsulfonic acid, and bromocamphorsulfonic acid, for which appropriate organic solvents (solvents in which the chiral sulfonic acid is soluble) would be an organic ester, such as methyl acetate, ethyl acetate, isopropyl acetate and butyl acetate; an organic ether, such as diethyl ether, methyl-t-butyl ether, tetrahydrofuran, dioxane and dimethoxyethane; acetonitrile; or mixtures thereof. Useful aromatic aldehydes include benzaldehyde, salicylaldehyde, 3,5-dichlorosalicyl-aldehyde and p-$NO_2$ benzaldehyde, the catalytic amount of which for effecting racemization of the unwanted isomer of the selected primary amine after crystallization of the desired isomer has occurred in the resolution mixture, is generally about 3 to 5 mole %, based on the calculated amount of mixture present.

The process is useful for primary amines with acidic α-hydrogens, including, for example, 3-amino-diazepin-2-ones, such as 3(R,S)-amino-1,3-dihydro-1-methyl-5-phenyl-2H-1,4-benzodiazepin-2-one, for which a key process intermediate in resolution-racemization process of the present invention is 3(S)-amino-1,3-dihydro-1-methyl-5-phenyl-2H-1,4-benzodiazepin-2-one, camphorsulfonic acid salt.

Specifically, the schematic of the process according to this invention may be represented by:

**Resolution - Racewization**

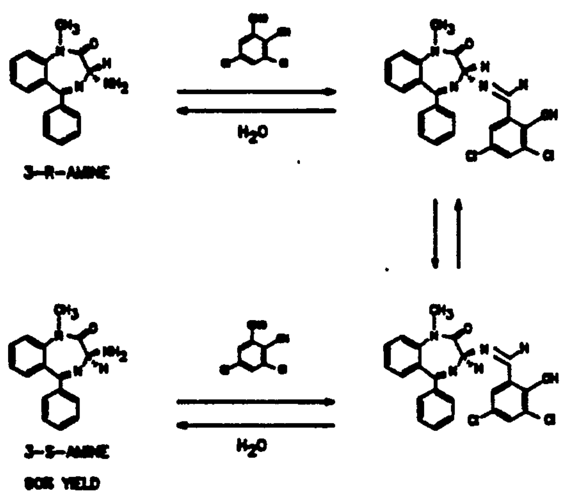


The racemic amine is resolved by selectively crystallizing the 3-S-amine as its (1S)-(+)-10-camphorsulfonic acid (CSA) salt. Treatment of a solution of (±) amine with 0.5 equivalent CSA and seeding the effects with crystals isolated from an oily mixture of the 3-amino-diazepin-2-ons and CSA in ethyl acetate-ether, in, for example, ethyl acetate, isopropyl acetate, or acetonitrile, results in the 3-S-amine CSA with greater than 99.5 % enantiomeric purity. Isolated yields are generally 40 - 42 %, with the mother liquors containing an approximately 90 : 10 ratio of the 3R : 3S amine, and the isolated 3-S-amine CSA salt appearing to be a hemi-hydrate. The addition of small amounts of water to the resolution mixture (KF range = 0.5 - 3.0 mg/ml) optimizes the crystallization.

The racemization of the unwanted 3-R-isomer is then achieved by addition of a catalytic amount (3-5 %) of an aromatic aldehyde, and an equilibrium concentration of imine is established. The acidity of the alpha proton is thus increased, allowing the chiral center to racemize at ambient temperature and basicity.

In the presence of 0.86 - 0.94 moles of CSA, the crystalline 3-S-amine CSA salt is essentially removed from the equilibrium system by virture of its insolubility, resulting in the 3-R-amine being continuously shunted through the imines to the desired 3-S-amine.

This combined resolution-racemization is easily run. The racemic amine is dissolved in isopropyl acetate and treated with 86 to 94 mole % CSA in $CH_3CN$ (with appropriate seeds of the desired end-product) to give generally greater than 40 % crystallization of the 3-S-amine CSA (by assay of the supernatant). The introduction of generally 3 to 5 mole % of 3,5-dichlorosalicylaldehyde to the reaction slurry then causes racemization with a $t_{1/2}$ of about six hours at 24 C.

After a total of about 36 hours, the product is isolated by filtration in approximately 83 % yield based on amine or approximately 97 % yield based on the limiting reagent, CSA. The rate of racemization does increase with temperature, but there is some accompanying decomposition, and if greater than 90 mole % CSA is charged, the rate of racemization slows, with the use of greater than or equal to 100 mole % CSA virtually preventing the racemization. In the event of a CSA overcharge, the addition of free amine will reinitiate the racemixation.

The following example is intended to further illustrate the invention, without limiting it.

**Example 1**

Preparation of 3(S)-amino-1,3-dihydro-1-methyl-5-phenyl-2H-1,4-benzodiazepin-2-one, camphorsulfonate salt

**Resolution**

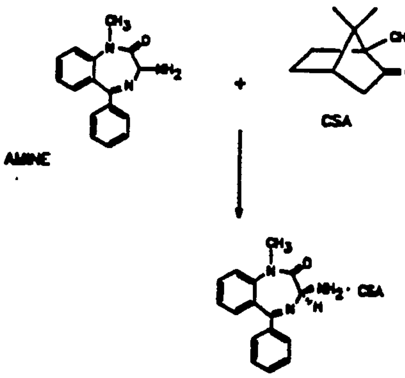

3 - R/S - AMINE

CSA

3 - S - AMINE CSA SALT

A solution of 1.367 moles of the racemic amine prepared according to Examples 183 and 184 from EPO Published Application 167 919 in isopropyl acetate (6000 ml) was assayed (HPLC, titration) and 86 mole % of (1S)-(+)-10-camphorsulfonic acid (CSA) (273 g. 1.17 moles) in acetonitrile (1600 ml), (i.e.: 0.75 M solution) was added, while maintaining the temperature $\leqslant$ 20°C, with the mixture being seeded with 1.0 g of 3-S amine CSA when ca. 50 % of the CSA had been charged. The white suspension was allowed to warm to 20 - 25°C and was aged for 4 hours, at which time the supernatant was assayed.

When 35 - 42 % of the amine (70 - 84 % of the 3-S-amine) was out of solution as the CSA salt (by assay of the supernatant), the mixture was treated with 7.8 g (0.04 mole, 3 mole %) of 3,5-dichlorosalicylaldehyde and the slurry was aged, with stirring, at 20 - 30°C for 36 hours. Upon completion of the resolution, the slurry was filtered and the cake washed with isopropyl acetate (3000 ml - as a 2000 ml slurry wash followed by a 1000 ml displacement wash). After a hexane wash (6000 ml) the product was vacuum dried (25°C). Yield 565 g (83 %).

HPLC: (wt %) $\geqslant$ 98 % as amine (corrected) (chiral) $\geqslant$ 99.8 % 3-S isomer.

**Claims**

1. A process for the resolution-racemization of primary amines with α-hydrogens, the pKa of which is less than or equal to fifteen, which comprises:

(a) adding 0.86 - 0.94 mole equivalent of a chiral sulfonic acid in an appropriate organic solvent and from one to 5 mole equivalent of water to a solution of a racemic mixture of a primary amine with an α-hydrogen to create a resolution mixture;

(b) seeding the resolution mixture with a crystalline diastereomeric salt derived from said primary amine and said chiral sulfonic acid;

(c) adding a catalytic amount of an aromatic aldehyde to the resolution mixture;

and

(d) filtering the resolution mixture to isolate the optically-pure diastereomeric salt of said primary amine.

2. A process according to Claim 1, wherein the primary amine is a 3-amino-diazepin-2-one.

3. A process according to Claim 2, wherein the chiral sulfonic acid is a camphorsulfonic acid or a bromocamphorsulfonic acid; the appropriate organic solvent is an organic ester, an organic ether, acetonitrile or a mixture thereof; the aromatic aldehyde is salicylaldehyde, 3,5-dichlorosalicylaldehyde, benzaldehyde or p-$NO_2$ benzaldehyde and the catalytic amount of said aromatic aldehyde is from about three to about five mole percent.

4. A process according to Claim 3, wherein the primary amine is 3(R,S)-amino-1,3-dihydro-1-methyl-5-phenyl-2H-1,4-benzodiazepin-2-one; the chiral sulfonic acid is (1S)-(+)-10-camphorsulfonic acid; the appropriate organic solvent is a mixture of isopropyl acetate and acetonitrile; and the aromatic aldehyde is 3,5-dichlorosalicylaldehyde.

5. 3(S)-amino-1,3-dihydro-1-methyl-5-phenyl-2H-1,4-benzodiazepin-2-one, (1S)-(+)-10-camphorsulfonate salt.

## Patentansprüche

1. Verfahren zur Aufspaltung/Razemisierung von primären Aminen mit α-Wasserstoffen, deren pKa weniger oder gleich 15 ist, durch

(a) Zugabe von 0,86 bis 0,94 Moläquivalenten einer chiralen Sulfonsäure in einem geeigneten organischen Lösungsmittel und von einem bis 5 Moläquivalenten Wasser zu einer Lösung einer razemischen Mischung eines primären Amins mit einem α-Wasserstoff zur Erzeugung einer Aufspaltungsmischung;

(b) Beimpfen der Aufspaltungsmischung mit einem von dem primären Amin und der chiralen Sulfonsäure abgeleiteten, kristallinen, diastereomeren Salz;

(c) Zugabe einer katalytischen Menge eines aromatischen Aldehyds zur Aufspaltungsmischung; und

(d) Filtern der Aufspaltungsmischung zur Isolierung des optisch reinen diasteromeren Salzes des primären Amins.

2. Verfahren nach Anspruch 1, worin das primäre Amin ein 3-Aminodiazepin-2-on ist.

3. Verfahren nach Anspruch 2, worin die chirale Sulfonsäure eine Kampfersulfonsäure oder eine Bromkampfersulfonsäure ist; das geeignete organische Lösungsmittel ein organischer Ester, ein organischer Ether, Acetonitril oder eine Mischung davon ist; der aromatische Aldehyd Salicylaldehyd, 3,5-Dichlorsalicylaldehyd, Benzaldehyd oder p-$NO_2$-Benzaldehyd ist und die katalytische Menge des aromatischen Aldehyds etwa 3 bis etwa 5 Mol-% beträgt.

4. Verfahren nach Anspruch 3, worin das primäre Amin 3(R,S)-Amino-1,3-dihydro-1-methyl-5-phenyl-2H-1,4-benzodiazepin-2-on ist; die chirale Sulfonsäure (1S)-(+)-10-Kampfersulfonsäure ist; das geeignete organische Lösungsmittel eine Mischung aus Isopropylacetat und Acetonitril ist; und der aromatische Aldehyd 3,5-Dichlorsalicylaldehyd ist.

5. (1S)-(+)-10-Kampfersulfonatsalz von 3(S)-Amino-1,3-dihydro-1-methyl-5-phenyl-2H-1,4-benzodiazepin-2-on.

## Revendications

1. Procédé de résolution-racémisation d'amines primaires comportant des α-hydrogènes, dont le pKa est inférieur ou égal à quinze, qui comprend:

(a) l'addition de 0,86 - 0,94 équivalents-moles d'un acide sulfonique chiral dans un solvant organique approprie et de 1 à 5 équivalents-moles d'eau à une solution d'un mélange racémique d'une amine primaire comportant un α-hydrogène pour créer un mélange de résolution;

(b) l'ensemencement du mélange de résolution avec un: sel diastéréoisomère cristallin dérivé de l'amine primaire et de l'acide sulfonique chiral;

(c) l'addition d'une quantité catalytique d'un aldéhyde aromatique au mélange de résolution; et

(d) la filtration du mélange de résolution pour isoler le sel diastéréoisomère optiquement pur de l'amine primaire.

2. Procédé selon la revendication 2, dans lequel l'amine primaire est une 3-aminodiazépine-2-one.

3. Procédé selon la revendication 2, dans lequel l'acide sulfonique chiral est un acide camphosulfonique ou un acide bromocamphosulfonique; le solvant organique approprié est un ester organique, un éther organique, l'acétonitrile ou un de leurs mélanges; l'aldéhyde aromatique est le salicylaldéhyde, le 3,5-dichlorosalicylaldéhyde, le benzaldéhyde ou le p-$NO_2$-benzaldéhyde, et la quantité catalytique de l'aldéhyde aromatique est d'environ 3 à environ 5 % en moles.

4. Procédé selon la revendication 3, dans lequel l'amine primaire est la 3(R,S)-amino-1,3-dihydro-1-méthyl-5-phényl-2H-1,4-benzodiazépine-2-one; l'acide sulfonique chiral est l'acide (1S)-(+)-10-camphosulfonique; le solvant organique approprié est un mélange d'acétate d'isopropyle et d'acétonitrile; et l'aldéhyde aromatique est le 3,5-dichlorosalicylaldéhyde.

5. Sel (1S)-(+)-10-camphosulfonate de 3(S)-amino-1,3-dihydro-1-méthyl-5-phényl-2H-1,4-benzodiazépine-2-one.